# EUROPÄISCHE PATENTSCHRIFT

(11) **EP 1 373 234 B1**
(45) Veröffentlichungstag und Bekanntmachung des Hinweises auf die Patenterteilung: **13.02.2008**
(21) Anmeldenummer: 02703436.2
(22) Anmeldetag: 19.03.2002
(51) Int. Cl.: C07D 295/20, A61K 31/495, A61P 35/00

(54) **UROKINASE-INHIBITOREN**
UROKINASE INHIBITORS
INHIBITEURS D'UROKINASE

(30) Priorität: 21.03.2001 WO PCT/CH01/00178
(43) Veröffentlichungstag der Anmeldung: 02.01.2004
(73) Patentinhaber: Wilex AG, 81675 München (DE)
(72) Erfinder: ZIEGLER, Hugo, CH-4108 Witterswil (CH)
(74) Vertreter: Weiss, Wolfgang
(86) Internationale Anmeldenummer: PCT/CH2002/000162
(87) Internationale Veröffentlichungsnummer: WO 2002/074756

(56) Entgegenhaltungen:
- WO-A-00/17158
- WO-A-96/05189
- A ZEGA ET AL: "Novel Thromibn inhibitors with azaphenylalanine scaffold" PHARMAZIE., Bd. 56, 1. September 2001 (2001-09-01), Seiten 683-685, XP002215735 VEB VERLAG VOLK UND GESUNDHEIT. BERLIN., DD ISSN: 0031-7144
- SANGSOO K ET AL: "Rational design of selective thrombin inhibitors" BIOORGANIC & MEDICINAL CHEMISTRY LETTERS, OXFORD, GB, Bd. 7, Nr. 7, 8. April 1997 (1997-04-08), Seiten 769-774, XP004136127 ISSN: 0960-894X
- YEONG S O ET AL: "Discovery of LB30057, a benzamidrazone-based selective oral thrombin inhibitor" BIOORGANIC & MEDICINAL CHEMISTRY LETTERS, OXFORD, GB, Bd. 8, Nr. 6, 17. März 1998 (1998-03-17), Seiten 631-634, XP004136935 ISSN: 0960-894X
- ZEGA A ET AL: "Design and structure-activity relationship of thrombin inhibitors with an azaphenylalanine scaffold: potency and selectivity enhancements via P2 optimization" BIOORGANIC & MEDICINAL CHEMISTRY, ELSEVIER SCIENCE LTD, GB, Nr. 9, 2001, Seiten 2745-2756, XP002196794 ISSN: 0968-0896
- JÖRG STÜRZEBECHER ET AL: "Synthesis and Structure-Activity Relationships of Potent Thrombin Inhibitors: Piperazides of Amidinophenylalanine" JOURNAL OF MEDICINAL CHEMISTRY, AMERICAN CHEMICAL SOCIETY. WASHINGTON, US, Bd. 19, Nr. 40, 12. September 1997 (1997-09-12), Seiten 3091-3099, XP002077904 ISSN: 0022-2623
- STURZEBECHER J ET AL: "3-Amidinophenylalanine-based Inhibitors of Urokinase" BIOORGANIC & MEDICINAL CHEMISTRY LETTERS, OXFORD, GB, Bd. 9, Nr. 21, 1. November 1999 (1999-11-01), Seiten 3147-3152, XP004181024 ISSN: 0960-894X

## Beschreibung

Bei der Ausbreitung und Metastasierung solider Tumoren spielen proteolytische Prozesse eine entscheidende Rolle. Zum Auf- und Abbau der Strukturen ihrer unmittelbaren Umgebung verfügen sie neben prokoagulatorischen Substanzen über Enzyme des Fibrinolyse-Systems. Obwohl die (patho)bio-chemischen Zusammenhänge noch nicht endgültig geklärt sind, kommen dem Plasminogenaktivator Urokinase und dem Urokinase-Rezeptor offenbar eine zentrale Bedeutung zu. Die Entwicklung von Hemmstoffen der Urokinase kann deshalb in erster Linie für die weitere Aufklärung der Rolle von Urokinase und dem Urokinase-Rezeptor bei verschiedenen Krankheiten, speziell bei der Tumorausbreitung und Metastasierung, von grossem Nutzen sein. Daneben stellen Urokinase-Inhibitoren potentielle Arzneimittel zur Beeinflussung der Tumor-Invasion dar.

Urokinase ist ein proteolytisches Enzym und gehört zur Gruppe der Trypsin-ähnlichen Enzyme, die in Proteinen und Peptiden die Bindungen der basischen Aminosäuren Arginin und Lysin spalten. Die meisten der heute bekannten Hemmstoffe besitzen deshalb eine stark basische Gruppe, z.B. eine Amidino-Funktion. Erste im mikromolaren Bereich wirksame Hemmstoffe der Urokinase wurden unter Bis-Benzamidinen und unter Verbindungen, die sich vom Naphthamidin ableiten, gefunden (J. Stürzebecher und F. Markwardt, Pharmazie 33, 599-602, 1978). Später wurden Verbindungen mit einer Guanidino-Funktion, wie Amiloride (J.-D. Vassalli und D. Belin, FEBS Lett. 214, 187-191, 1987) und Phenylguanidine (H. Yang et al., J. Med. Chem. 33, 2956-2961, 1990) beschrieben, welche Urokinase ebenfalls mit mikromolaren Kᵢ-Werten hemmen. Als sehr wirksame Inhibitoren (Kᵢ bei 0,2µmol/l) wurden Benzothiophen-2-carboxamidine beschrieben (M. J. Towle et al., Cancer.Res. 53, 2553-2559, 1993).

Nα-Arylsulfonylierte und Nα-arylsulfonyl-aminoacylierte Derivate des 3-Amidinophenylalanins sind als selektive Hemmstoffe des Thrombins (F. Markwardt et al., Thromb. Res. 17, 425-431, 1980) bzw. von Gerinnungsfaktor Xa (J. Stürzebecher et al., Thromb. Res. 54, 245-252, 1989) bzw. der Urokinase (P. Wikstroem et al., WO 00/17158 und J. Stürzebecher et al., WO 00/04954) bekannt. Bei der Variation der Phenylalanin-Gruppe wurde nun gefunden, dass der Austausch der CH-Funktion des Phenylalanins durch ein Stickstoffatom unter gleichzeitiger Einführung eines (Hetero-) Aryl-Restes am Sulfonyl-Teil die Affinität zu Urokinase ganz entscheidend erhöht. Deshalb stellen α-(3-Amidinobenzyl)-β-(hetero-)
arylsulfonyl-hydrazide und die durch Austausch der Amidino-Funktion durch einen Amidoxim-Rest zugänglichen Prodrugs (vgl. D. Baucke et al., WO 00/61577) neue Gruppen von Urokinase-Hemmstoffen dar.

Die vorliegende Erfindung betrifft neue Urokinase-Inhibitoren der allgemeinen Formel I 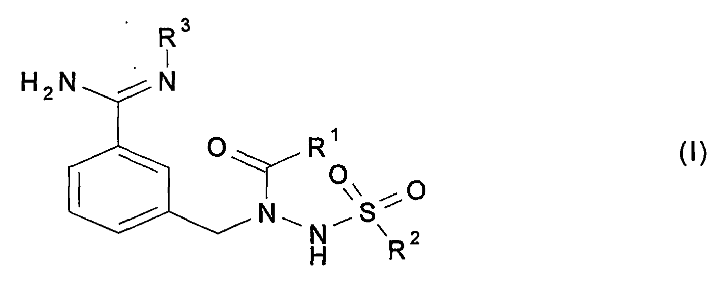 die bezüglich R¹ als Racemate sowie als L- bzw. D-konfigurierte Verbindungen und als E/Z-Gemische sowie als E- bzw. Z-Isomere vorliegen und in denen
- R¹: (a) OH oder gegebenenfalls mit Aryl substituiertes (C₁-C₈)-Alkoxy oder (C₃-C₈)-Cycloalkyloxy bedeutet; oder
(b) eine Gruppe der Formel -N(R⁴R⁵) bedeutet, in welcher R⁴ und R⁵ unabhängig voneinander Wasserstoff, (C₁-C₈)-Alkyl, mit gegebenenfalls 1- bis 3-fach identisch oder unterschiedlich substituiertem Aryl substituiertes (C₁-C₈)-Alkyl, (C₃-C₈)-Cycloalkyl, (C₃-C₈)-Cycloalkyl-(C₁-C₄)-alkyl, NH₂, NHR⁶ oder NR⁶R⁷ bedeuten, wobei R⁶ und R⁷ unabhängig voneinander (C₁-C₈)-Alkyl, (C₃-C₈)-Cycloalkyl, gegebenenfalls 1- bis 3-fach identisch oder unterschiedlich substituiertes Aryl, gegebenenfalls 1-bis 3-fach identisch oder unterschiedlich substituiertes Heteroaryl bedeuten, oder R⁶ und R⁷ zusammen mit dem Stickstoffatom, an das sie gebunden sind, einen Thiazolin-, Thiazolidin-, Oxazolin-, Oxazolidin- oder Morpholinring bedeuten, welcher gegebenenfalls 1- bis 3-fach identisch oder unterschiedlich substituiert sein kann; oder
(c) eine Gruppe der Formel 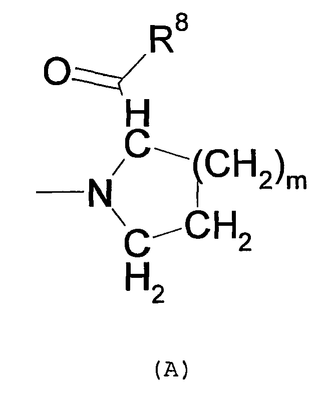 bedeutet, in welcher m die Zahl 1 oder 2 bedeutet und in welcher eine der Methylengruppen mit einem Hydroxyl-, Carboxyl- oder (C₁-C₈)-Alkylrest oder einem mit gegebenenfalls 1- bis 3-fach identisch oder unterschiedlich substituiertem Aryl substituierten (C₁-C₈)-Alkylrest substituiert sein kann und R⁸ eine der oben unter (a) und (b) angegebenen Bedeutungen von R¹ hat, wobei in 2,3- oder 3,4-Stellung, bezogen auf das Stickstoffatom, gegebenenfalls ein weiterer aromatischer oder cycloaliphatischer Ring ankondensiert ist; oder
(d) eine Gruppe der Formel 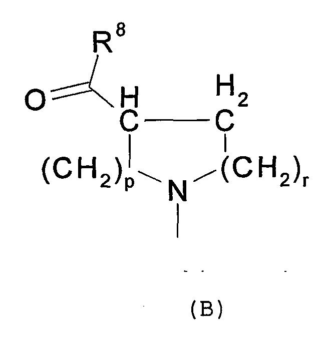 bedeutet, in welcher p und r unabhängig voneinander 1 oder 2 sind und in welcher eine der Methylengruppen mit einem Hydroxyl-, Carboxyl-, (C₁-C₈)-Alkyl- oder einem mit gegebenenfalls 1- bis 3-fach identisch oder unterschiedlich substituiertem Aryl substituierten (C₁-C₈)-Alkylrest substituiert sein kann und R⁸ eine der oben unter (a) und (b) angegebenen Bedeutungen von R¹ hat, wobei in 2,3- oder 3,4-Stellung, bezogen auf das Stickstoffatom, gegebenenfalls ein weiterer aromatischer oder cycloaliphatischer Ring ankondensiert ist; oder
(e) eine Piperidylgruppe bedeutet, die gegebenenfalls in einer der Stellungen 2, 3 und 4 mit einem (C₁-C₈)-Alkylcarbonyl-, einem (C₁-C₈)-Alkoxycarbonyl-, einem (C₁-C₄)-Alkyl- oder einem Hydroxylrest substituiert ist, wobei in 2,3- oder 3,4-Stellung, bezogen auf das Stickstoffatom, gegebenenfalls ein weiterer aromatischer oder cycloaliphatischer Ring ankondensiert ist; oder
(f) eine Gruppe der Formel 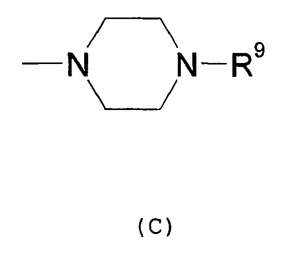 bedeutet, worin R⁹ H, (C₁-C₈)-Alkyl, (C₂-C₈)-Alkenyl, (C₂-C₈)-Alkinyl, Formyl, (C₁-C₈)-Alkylcarbonyl,C₂-C₈)-Alkenylcarbonyl, (C₂-C₈) -Alkinylcarbonyl, Cyano, (C₁-C₈)-Alkoxycarbonyl, (C₂-C₈)-Alkenyloxycarbonyl, (C₂-C₈)-Alkinyloxycarbonyl, (C₃-C₈) -Cycloalkyl, (C₃-C₈) Cycloalkylcarbonyl, (C₃-C₈) -Cycloalkyloxycarbonyl; (C₃-C₈)-Heterocyclyl, (C₃-C₈) Heterocyclylcarbonyl, (C₃-C₈)-Heterocyclyloxycarbonyl mit jeweils 1 bis 3 Heteroatomen; Aryl, Arylcarbonyl, Aryloxycarbonyl; Heteroaryl, Heteroarylcarbonyl, Heteroaryloxycarbonyl, mit jeweils 1 bis 3 Heteroatomen; einen Carbonsäureamidrest der Formel -CON(R⁴R⁵) oder einen Thiocarbonsäureamidrest der Formel -CSN(R⁴R⁵) bedeutet, in welchen R⁴ und R⁵ unabhängig voneinander Wasserstoff, (C₁-C₈)-Alkyl, (C₂-C₈)-Alkenyl, (C₂-C₈)-Alkinyl, (C₃-C₈) -Cycloalkyl, Aryl, oder Heteroaryl bedeuten oder R⁴ und R⁵ zusammen mit dem Stickstoffatom, an das sie gebunden sind, einen 5- bis 7-gliedrigen Ring bedeuten, der 1 bis 2 weitere Heteroatome (N, O, S) enthalten kann und der gegebenenfalls 1- bis 3-fach identisch oder unterschiedlich substituiert sein kann oder einen -SO₂Y-Rest bedeutet, in welchem Y (C₁-C₈)-Alkyl, (C₂-C₈)-Alkenyl, (C₂-C₈)-Alkinyl, (C₃-C₈) -Cycloalkyl, Aryl, Heteroaryl, Aryloxy oder N(R⁴R⁵) bedeutet; wobei alle Alkyl- Alkenyl- und Alkinylreste der Alkyl, Alkenyl bzw Alkinyl enthaltenden Gruppen 1- bis 3-fach identisch oder unterschiedlich substituiert und alle Cycloalkyl-, Heterocyclyl-, Aryl- und Heteroarylreste der Cycloalkyl, Heterocyclyl, Aryl bzw Heteroaryl enthaltenden Gruppen 1- bis 3-fach identisch oder unterschiedlich substituiert sein können;
- R²: einen gegebenenfalls 1- bis 3-fach identisch oder unterschiedlich substituierten Aryl- oder Heteroarylrest oder einen Campherrest bedeutet; und
- R³: Wasserstoff, Amino oder Hydroxy bedeutet;
wobei diese Verbindungen sowohl als freie Basen als auch als Salze mit Mineralsäuren oder als Salze mit organischen Säuren vorliegen können.

In der Regel liegen die Verbindungen der Formel I als Salze mit Mineralsäuren, bevorzugt als Hydrochloride, oder als Salze mit organischen Säuren vor.

Die Verbindungen der Formel I und deren Salze können erfindungsgemäss dadurch hergestellt werden, dass man
(a) zur Herstellung einer Verbindung der Formel I, worin R³ Wasserstoff oder Amino bedeutet, eine Cyanoverbindung der Formel 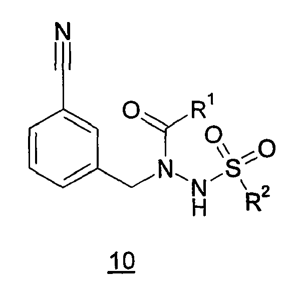 worin R¹ und R² obige Bedeutung besitzen, durch Addition von H₂S an die Cyangruppe in das entsprechende Thioamid überführt, aus welcher durch S-Alkylierung ein Thioimidoester erhalten wir, welcher anschliessend durch Behandlung mit Ammoniak oder Hydrazin oder einem Salz davon in die Amidino- bzw. Amidrazonverbindung übergeführt wird; oder
(b) zur Herstellung einer Verbindung der Formel I, worin R³ Wasserstoff oder Amino bedeutet, eine Cyanoverbindung der Formel 10 durch saure Alkoholyse in ein Säureadditionssalz des entsprechenden Imidoesters überführt, dessen Umsetzung mit Ammoniak oder Hydrazin zur Amidino- bzw. Amidrazonverbindung führt; oder
(c) zur Herstellung einer Verbindung der Formel I, worin R³ Wasserstoff bedeutet, eine Verbindung der Formel I, worin R³ OH bedeutet, oder eine entsprechende Alkanoyloxyverbindung, reduziert; oder
(d) zur Herstellung einer Verbindung der Formel I, worin R³ OH oder NH₂ bedeutet, eine Cyanoverbindung der Formel 10 mittels Hydroxylamin oder Hydrazin in die entsprechende Hydroxyamidin- bzw. Amidrazonverbindung überführt; und
(e) erwünschtenfalls eine erhaltene Verbindung der Formel I in ein Säureadditionssalz überführt und/oder ein erhaltenes Säureadditionssalz in die entsprechende Verbindung der Formel I oder in ein anderes Säureadditionssalz überführt.

Die Ausgangsprodukte der oben definierten Formel 10 sind ebenfalls Gegenstand der vorliegenden Erfindung.

Der Ausdruck "Alkyl", für sich allein genommen oder als Strukturelement für Alkyl enthaltende Gruppen, bezeichnet gesättigte Kohlenwasserstoffreste, welche geradkettig oder verzweigt sein können. Der Ausdruck "Cycloalkyl", ebenfalls für sich allein genommen oder als Strukturelement für Cycloalkyl enthaltende Gruppen, bezeichnet zyklische gesättigte Kohlenwasserstoffreste. Die Ausdrücke "Alkenyl" und "Alkinyl", wiederum für sich allein genommen oder als Strukturelemente für Alkenyl bzw. Alkinyl enthaltende Gruppen, bezeichnen geradkettige oder verzweigte, mindestens eine C-C-Doppel- bzw. - Dreifachbindung enthaltende Kohlenwasserstoffreste. Die Ausdrücke "Alkoxy" und "Cycloalkyloxy" bezeichnen über eine Sauerstoffgruppe verknüpfte Alkyl- bzw. Cycloalkylgruppen im Sinne der obigen Definitionen von "Alkyl" bzw. "Cycloalkyl".

Beispiele für Alkyl - als Gruppe per se und als Strukturelement für Alkyl enthaltende Gruppen - sind Methyl, Ethyl, Propyl, n-Butyl, n-Pentyl, n-Hexyl und n-Octyl als unverzweigte und Isopropyl, tert.Butyl, Isobutyl, sec.Butyl und Isoamyl als verzweigte Reste. Bevorzugt sind Methyl und Ethyl. Beispiele für Cycloalkyl - als Gruppe per se und als Strukturelement für Cycloalkyl enthaltende Gruppen - sind Cyclopropyl, Cyclobutyl, Cyclopentyl und Cyclohexyl. Beispiele für Alkenyl - als Gruppe per se und als Strukturelement für Alkenyl enthaltende Gruppen - sind unter anderem Vinyl, 1-Methylvinyl, Allyl, 1-Butenyl und 2-Hexenyl als unverzweigte und Isopropenyl als verzweigte Reste. Beispiele für Alkinyl - als Gruppe per se und als Strukturelement für Alkinyl enthaltende Gruppen - sind Propargyl, 2-Butinyl oder 5-Hexinyl als unverzweigte und 2-Ethinylpropyl oder 2-Propargylisopropyl als verzweigte Reste.

Der Ausdruck "Aryl" bezeichnet ein- zwei- oder mehrkernige aromatische Kohlenwasserstoffreste, beispielsweise Phenyl oder Naphthyl, bevorzugt Phenyl.

Der Ausdruck "Heteroaryl", für sich allein oder als Strukturelement für Heteroaryl enthaltende Gruppen, bezeichnet 5- bis 11-gliedrige aromatische Systeme bestehend aus einem oder zwei Ringen, in denen 1 bis 3 Glieder Heteroatome sind, ausgewählt aus Sauerstoff, Schwefel und Stickstoff. 1 bis 2 Benzolringe können an den Heterocyclus ankondensiert sein. Beispiele sind Pyidyl, Pyrimidinyl, Pyridazinyl, Pyrazinyl, 1,3,5-Triazinyl, Chinolinyl, Isochonolinyl Chinoxalinyl, Chinazolinyl, Phthalazinyl, Pyrrolyl, Pyrazolinyl, Imidazolinyl, 1,2,4-Triazolinyl, Tetrazolinyl, Furyl, Thienyl, Oxazolinyl, Thiazolinyl, Isothiazolinyl, Benzoxazolyl, Benzothienyl, Indolyl, Benzimidazolyl. Indazolyl, Benzotriazolyl und Benzothiazolyl, wobei die Verknüpfung entweder am Heteroteil oder am Benzoteil und bei den π-Überschussheteroaromaten über den Stickstoff oder über einen beliebigen Kohlenstoff erfolgen kann.

Der Ausdruck "Heterocyclyl", für sich allein und als Strukturelement für Heterocyclyl enthaltende Gruppen, bezeichnet einen 3- bis 8-gliedrigen, 1 bis 3 Heteroatome (ausgewählt aus O, S und N) enthaltenden nicht aromatischen Ring, an den ein Benzolring ankondensiert sein kann. Beispiele sind Oxiranyl, Pyrrolidinyl, Piperidinyl, Piperazinyl, Morpholinyl, Azepinyl, Oxazolidinyl, Oxazolinyl, Thiazolidinyl, Thiazolinyl und 1,2,3,4-Tetrahydrochinolinyl.

Substituenten der gegebenenfalls substituierten Aryl- und Heteroarylgruppen sind z.B. Halogen, C₁-C₆-Alkyl, C₁-C₆-Halogenalkyl, Hydroxy, C₁-C₆-Alkoxy, C₁-C₆-Halogenalkoxy, C₁-C₆-Alkenyl, C₂-C₆-Alkenyloxy, C₂-C₆-Alkinyl, C₃-C₆-Alkinyloxy, C₁-C₆-Alkoxycarbonyl, CN, OCN, Nitro, Amino, C₁-C₆-Alkylamino, Di-C₁-C₆-alkylamino, Aminocarbonyl, C₁-C₆-Alkylaminocarbonyl, Di-C₁-C₆-alkylaminocarbonyl, C₁-C₆-Alkylthio, C₁-C₆-Alkylsulfoxyl, C₁-C₆-Alkylsulfonyl, C₃-C₆-Cycloalkyl, ggf. substituiertes Benzyl, ggf. substituiertes Phenyl, ggf. substituiertes Phenoxy oder ggf. substituiertes Phenylcarbonyl, wobei die oben erwähnten aromatischen Ringe substituiert sein können mit 1 bis 3 identischen oder unterschiedlichen Substituenten, ausgewählt aus der Gruppe bestehend aus Halogen, Cyano, Nitro, C₁-C₆-Alkyl, C₁-C₆-Halogenalkyl, C₁-C₆-Alkoxy, C₁-C₆-Halogenalkoxy, Hydroxy, Amino, C₁-C₆-Alkylamino, Di-C₁-C₆-alkylamino und C₁-C₆-Alkoxycarbonyl.

Substituenten der ggf. substituierten, durch NR⁴R⁵ und NR⁶R⁷ gebildeten 5- bis 7-gliedrigen Ringe und der Heterocyclylgruppen sind z.B. Halogen, C₁-C₆-Alkyl, C₁-C₆-Halogenalkyl, Hydroxy, C₁-C₆-Alkoxy, CN, OCN, Nitro, Amino, C₁-C₆-Alkylamino, Di-C₁-C₆-alkylamino, C₁-C₆-Alkylthio, C₁-C₆-Alkylsulfoxyl, C₁-C₆-Alkylsulfonyl, C₃-C₆-Cycloalkyl, ggf. substituiertes Benzyl, ggf. substituiertes Phenyl oder ggf. substituiertes Phenoxy, wobei die oben erwähnten aromatischen Ringe substituiert sein können mit 1 bis 3 identischen oder unterschiedlichen Substituenten, ausgewählt aus der Gruppe bestehend aus Halogen, Cyano, Nitro, C₁-C₆-Alkyl, C₁-C₆-Halogenalkyl, C₁-C₆-Alkoxy, C₁-C₆-Halogenalkoxy, Hydroxy, Amino, C₁-C₆-Alkylamino, Di-C₁-C₆-alkylamino und C₁-C₆-Alkoxycarbonyl.

Substituenten der ggf. substituierten Alkyl-, Alkenyl- und Alkinylreste und der diese Reste enthaltenden Gruppen sind beispielsweise Halogen, Hydroxy, C₁-C₆-Alkoxy, Halogen-C₁-C₆-alkoxy, C₁-C₆-Alkoxycarbonyl, gegebenenfalls substituiertes Phenyl und Cyano.

Der am heterocyclischen Rest der Formel (A) oder (B) oder am Piperidinrest gegebenenfalls ankondensierte weitere Ring ist vorzugsweise ein Benzol- oder Cyclohexanring.

Halogen ist Fluor, Chlor, Brom und Jod, vorzugsweise Fluor und Chlor. Halogenalkyl- und Halogenalkoxyreste, welche durch mehr als ein Halogenatom substituiert sind, können identische oder unterschiedliche Halogenatome tragen.

Unter den Verbindungen der Formel I sind diejenigen bevorzugt, worin R¹ OH, gegebenenfalls mit Aryl substituiertes (C₁-C₈)-Alkoxy,(C₃-C₈)-Cycloalkyloxy oder eine Gruppe der Formel -NR⁴R⁵, (B) oder (C) bedeutet. Besonders bevorzugt sind diejenigen Verbindungen der Formel I, worin R¹ eine Gruppe der Formel (C) bedeutet, worin R⁹ Formyl, (C₁-C₈)-Alkylcarbonyl, (C₂-C₈)-Alkenylcarbonyl, (C₂-C₈)-Alkinylcarbonyl, (C₁-C₈)-Alkoxycarbonyl, (C₂-C₈)-Alkenyloxycarbonyl, (C₂-C₈)-Alkinyloxycarbonyl oder einen Carbonsäureamidrest der Formel -CON(R⁴R⁵) bedeutet, wobei R⁴ und R⁵ je Wasserstoff, (C₁-C₈)-Alkyl, (C₂-C₈) -Alkenyl oder (C₂-C₈) - Alkinyl bedeuten, insbesondere solche, worin R⁹ Methoxycarbonyl, Ethoxycarbonyl, Benzyloxycarbonyl, Dimethylaminocarbonyl oder Acetyl bedeutet.

R² bedeutet beispielsweise Phenyl, 4-Tolyl, 2,4,6-Trimethylphenyl, 2,4,6-Triisopropylphenyl, 4-Methoxy-2,3,6-trimethylphenyl, 2,2-Dimethyl-6-methoxychromanyl, 2,2,5,7,8-Pentamethylchromanyl, Anthrachinonyl, 1-Naphthyl, 2-Naphthyl, 5-(Dimethylamino)-naphthyl, Chinolyl, Isochinolyl, oder einen Campherrest, vorzugsweise 2,4,6-Triisopropylphenyl.

R³ bedeutet vorzugsweise Wasserstoff.

Die Herstellung der Verbindungen der Formel I und der hierfür benötigten Ausgangsprodukte der Formel 10 wird in dem nachstehenden Schema 1 näher erläutert.

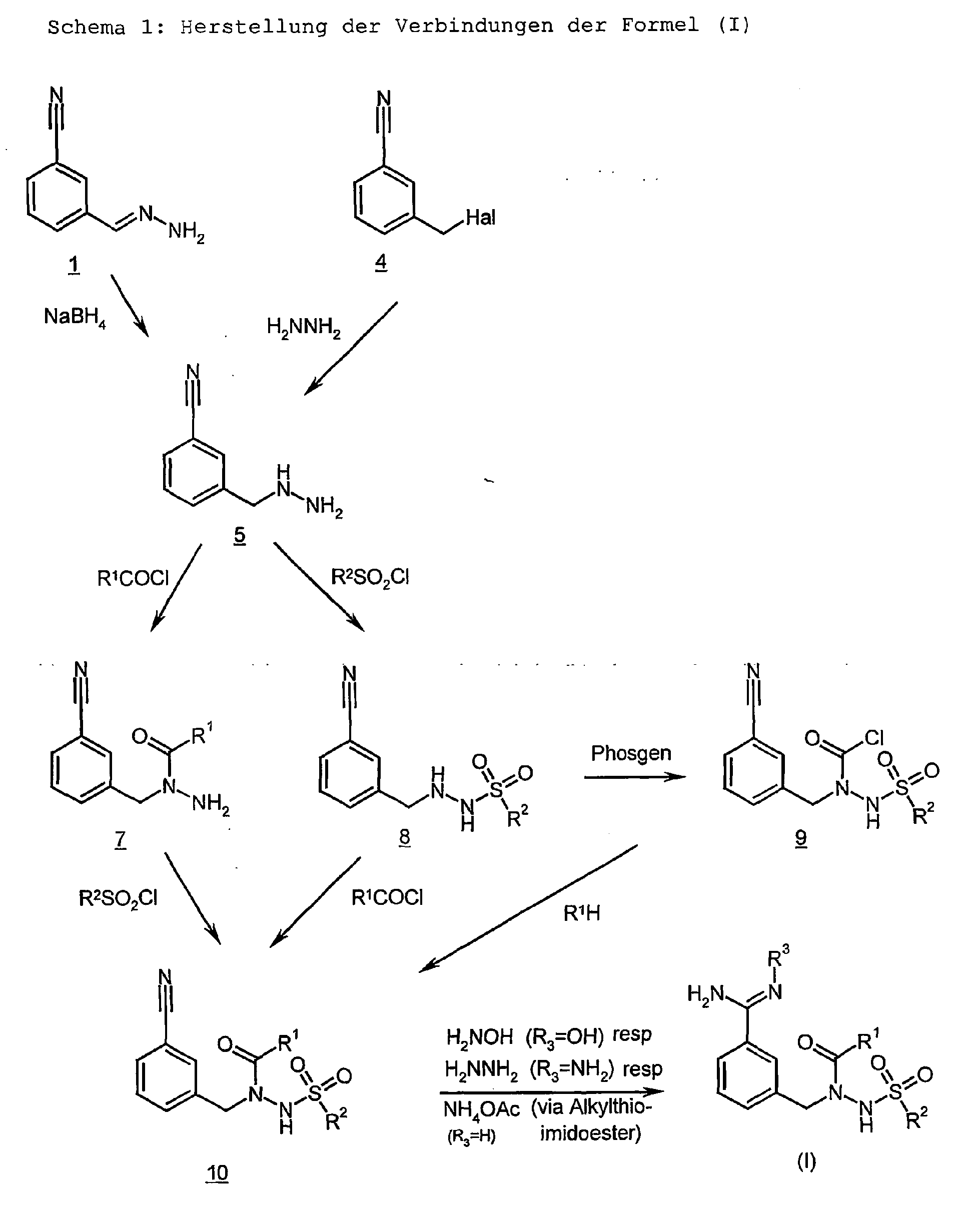

Die Verbindungen der allgemeinen Formel I mit Amidinstruktur (R³ = H) sind aus den Cyanoverbindungen 10 in an sich bekannter Weise erhältlich, wobei in der Regel durch Addition von H₂S an die Cyangruppe zunächst die entsprechenden Thioamide erhalten werden, die durch S-Alkylierung, z.B. S-Methylierung mit Methyliodid, in die entsprechenden Thioimidoester und anschliessend durch Behandlung mit Ammoniak oder einem Salz davon, z.B. mit Ammoniumacetat in alkoholischer Lösung, in die entsprechenden Amidinoverbindungen übergeführt werden (vgl. z.B. WO 94/18185, S. 23f; WO 00/17158, S. 5ff). Ausserdem können gegebenenfalls aus den Cyanoverbindungender Formel 10 durch saure Alkoholyse, z.B. mit Methanol oder Ethanol in Gegenwart von HCl-Gas und in bestimmten Fällen eines inerten Lösungsmittels, die entsprechenden Imidoestersalze, z.B. also die Imidoesterhydrochloride, hergestellt werden, deren Umsetzung mit Ammoniak, z.B. in alkoholischer Lösung, zu den entsprechenden Amidinoverbindungen führt. Weiterhin lassen sich diese Amidinoverbindungen durch Reduktion, beispielsweise durch Palladium-katalysierte Hydrierung, der entsprechenden Hydroxyamidinverbindungen (Formel I, R³ = OH) oder deren acylierten Derivate, wie zum Beispiel der entsprechenden Acetyloxyamidinverbindungen, erhalten.

Die Verbindungen der allgemeinen Formel I mit Hydroxyamidinstruktur (R³ = OH) sind aus den Cyanoverbindungen der Formel 10 mittels Hydroxylamin, z.B. durch Umsetzung mit einer alkoholischen Hydroxylamin-Lösung, zugänglich (vgl. z.B. WO 00/61577, S. 36).

Die Verbindungen der allgemeinen Formel I mit Amidrazonstruktur (R³ = NH₂) sind aus den Cyanverbindungen über die Thioimidoester oder die Imidoester, wie oben erwähnt, oder direkt durch Reaktion mit einer alkoholischen Hydrazin-Lösung zugänglich (vgl. z.B. Pavlov, P. A.; Kul'nevich, V. G; Khim. Geterotsikl. Soedin. (1986), (2), 181-186).

Eine Cyanoverbindung der Formel 10 lässt sich herstellen, indem
(aa) ein Hydrazid der Formel 7 mit einem Sulfochlorid der allgemeinen Formel R²SO₂Cl oder einem Sulfonsäureanhydrid der allgemeinen Formel (R²SO₂)₂O, worin R² die eingangs genannte Bedeutung hat, zur Reaktion gebracht wird; oder
(bb) ein Sulfonylhydrazid der Formel 8 mit einem Säurechlorid der allgemeinen Formel R¹COCl oder einem Anhydrid der allgemeinen Formel (R¹CO)₂O, worin R¹ die eingangs genannte Bedeutungen hat, umgesetzt wird; oder
(cc) ein Sulfonyl-chlorcarbonyl-hydrazid der Formel 9 mit einem Nukleophil der allgemeinen Formel R¹H, worin R¹ die eingangs genannte.Bedeutung hat, umgesetzt wird.

Die obigen Reaktionstypen (aa) bis (cc) sind an sich bekannt, und die Cyanoverbindung 10 lässt sich analog dazu herstellen.

Ein Sulfonyl-chlorcarbonyl-hydrazid der Formel 9 kann hergestellt werden, indem ein Sulfonylhydrazid der Formel 8 mit Phosgen, Diphosgen oder Triphosgen zur Reaktion gebracht wird, z.B. in Analogie zu der in J. Org. Chem. 41, 3763 (1976) beschriebenen Methode.

Die Verbindungen der allgemeinen Formeln 7 und 8 lassen sich, wie in Schema 1 gezeigt, nach an sich bekannten Wegen und Methoden synthetisieren.

Die erfindungsgemässen Urokinaseinhibitoren eignen sich zur Anwendung als therapeutische Wirkstoffe oder für diagnostische Zecke. Sie können zu Arzneimitteln, enthaltend eine Verbindung der Formel I oder ein Salz davon und gegebenenfalls mindestens einen für ein Arzneimittel geeigneten Hilfsstoff, bzw. zu diagnostischen Mitteln, enthaltend eine Verbindung der Formel I oder ein Salz davon und gegebenenfalls mindestens einen für ein diagnostisches Mittel geeigneten Hilfsstoff, verarbeitet werden. Die Verbindungen der Formel I oder deren Salze bzw. die sie enthaltenden Mittel können für die Diagnostik, Therapie und Prävention von Urokinase- oder Urokinaserezeptor-assoziierten Krankheiten verwendet werden, so für die Tumorbekämpfung, denn sie sind in der Lage, hocheffizient das Wachstum und/oder die Ausbreitung von malignen Tumoren zu hemmen, z.B. die Tumorausbreitung beim Pankreaskarzinom, das Tumorwachstum des Mammakarzinoms sowie die Metastasierung von Tumoren. Insbesondere eignen sie sich für die Bekämpfung von Mammakarzinomen, Pankreaskarzinomen und der Metastasenbildung, sowie für die Bekämpfung von Pemphigus vulgaris.

Die erwähnten Mittel können eine oder mehrere der Verbindungen der Formel I oder Salze davon in Kombination mit mindestens einer weiteren pharmakologisch wirksamen Substanz enthalten, z.B. mit mindestens einer Radiomarkierung und/oder mindestens einer zytotoxischen Substanz.

Bei den Arzneimitteln, enthaltend eine Verbindung der Formel I oder ein Salz davon, handelt es sich um oral, topisch, rektal oder parenteral, z.B. subkutan oder intravenös, verabreichbare Arzneimittel. Diese Arzneimittel können in Form von Tabletten, Dragees, Kapseln, Pellets, Suppositorien, Lösungen, Pflastern oder anderen transdermalen Systemen vorliegt.

Die erfindungsgemässen Verbindungen und Salze können gegebenenfalls zusammen mit anderen Antitumormitteln oder mit anderen Behandlungsarten, z.B. Bestrahlung oder chirurgischen Eingriffen, eingesetzt werden.

Bemerkenswert ist, dass die erfindungsgemässen Verbindungen und Salze die Blutgerinnung nur geringfügig beeinflussen, da sie für eine effektive Hemmung von Thrombin und Faktor Xa zu hohe Kᵢ-Werte haben.

Die nachfolgenden Beispiele sollen die Erfindung näher illustrieren, ihren Umfang jedoch in keiner Weise beschränken.

### Herstellungsbeispiele

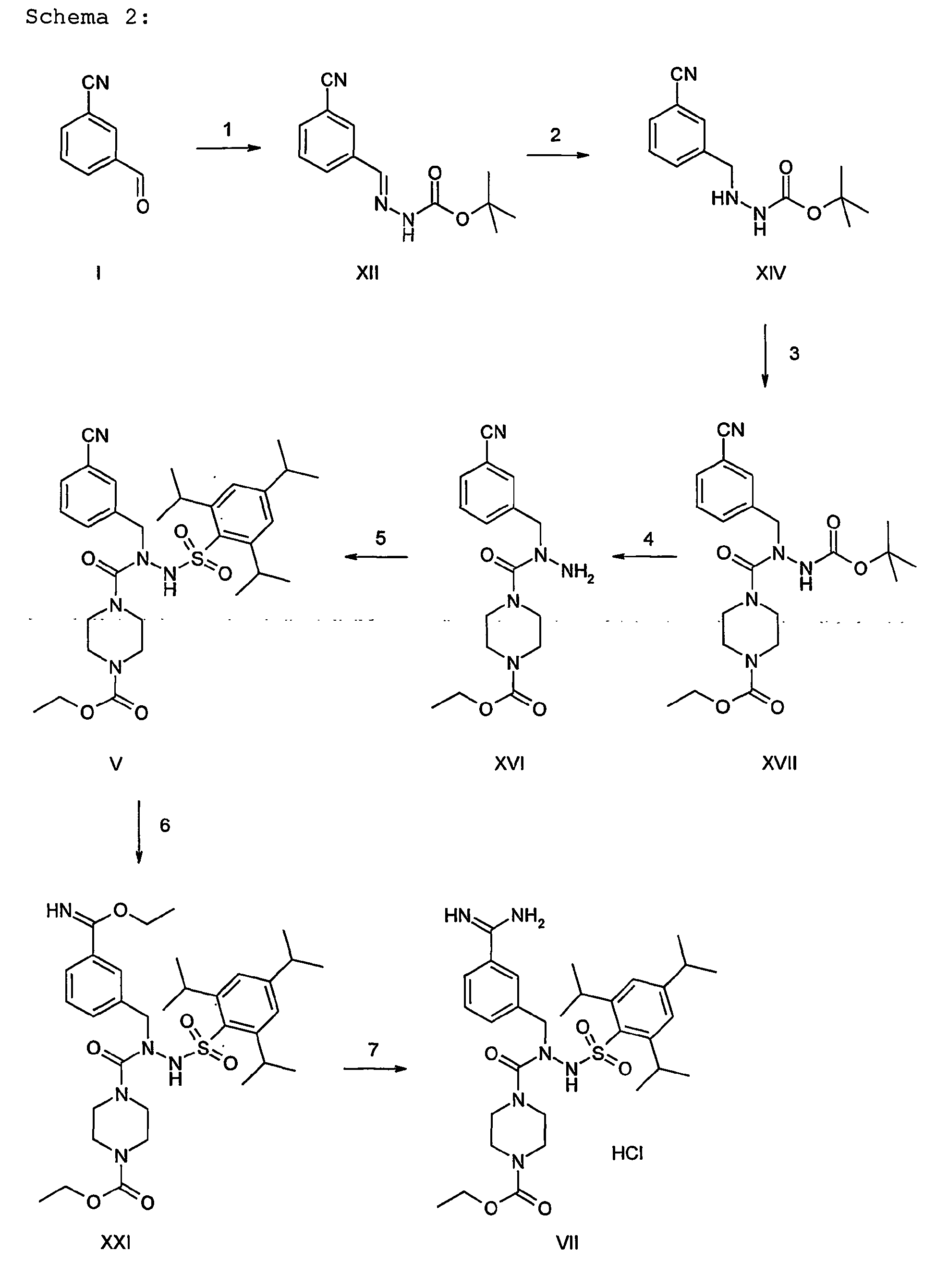

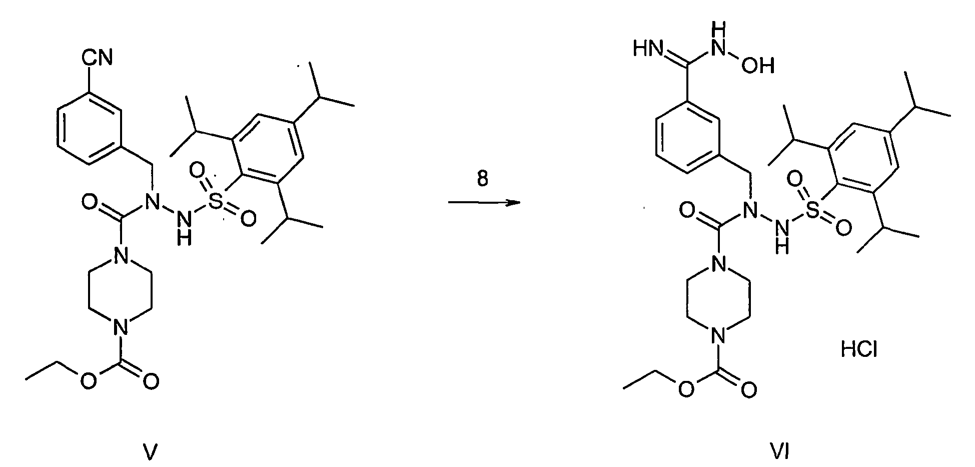

Die Verbindungen gemäss Schema 2 wurden wie folgt hergestellt:
3-Cyanobenzaldehyd-(1,1-dimethyl)-ethoxycarbonyl-hydrazon XII: 1 mmol 3-Cyanobenzaldehyd und 1mmol t-Butylcarbazat werden in 2.5 ml Essigester zum Rückfluss erhitzt. Die entstandene Suspension wird auf 0°C gekühlt, und der Festkörper abfiltriert. Die Mutterlauge wird aufkonzentriert, und die entstandenen Kristalle werden erneut abfiltriert. Ausbeute 74%.
   ¹H-NMR (500 MHz, dmso): 1.48 (s, 9H), 7.62 (t, 1H), 7.83 (dt, 1H), 7.95 (dt, 1H), 8.01 (t, 1H), 8.04 (s, 1H), 8.5 (breit, 1H). ¹³C-NMR: 28.5 (3C), 80.1, 112.3, 118.8, 130.34 (2), 130.36, 130.8, 132.9, 136.3, 152.6.
1-(3-Cyanobenzyl)-2-[(1,1-dimethyl)-ethoxycarbonyl]-hydrazin XIV: 1g XII wird in 10 ml Ethanol mit 0.1 mol% Pd/C bei Raumtemperatur hydriert. Das Reaktionsgemisch wird über eine Nutsche mit Celite abfiltriert, und der Filterkuchen wird mit Ethanol nachgewaschen. Das Filtrat wird eingeengt, und der Rückstand wird getrocknet. Ausbeute: 98%.
   ¹H-NMR (500 MHz, dmso): 1.39 (s, 9H), 3.95 (s, 2H), 5.10 (s, 1H), 7.51 (t, 1H), 7.64 (dt, 1H), 7.71 (dt, 1H), 7.80 (s, 1H), 8.5 (breit, 1H).
   ¹³C-NMR: 28.5 (3C), 46.0, 78.7, 111.4, 119.4, 129.5 (2C), 130.8 (2C), 144.6, 152.6.
1-Ethoxycarbonyl-4-[1-(3-cyanobenzyl)-2-((1,1-dimethyl)-ethoxycarbonyl)-1-hydrazino-carbonyl]-piperazin XVII: Zu einer Lösung von 1 mmol Triphosgen in 2 ml CH₂Cl₂ wird bei Raumtemperatur eine Lösung von 2 mmol XIV und 3 mmol Diisopropylethylamin (DIPEA) in 4 ml CH₂Cl₂ zugetropft. Nach 15 Minuten wird eine Lösung von 2 mmol Piperazin-1-carbonsäureethylester und 3 mmol DIPEA in 4 ml CH₂Cl₂ zugetropft. Das Gemisch wird 1 Stunde gerührt, mit 10 %iger Zitronensäure und gesättigter NaHCO₃-Lösung gewaschen, über Na₂SO₄ getrocknet und anschliessend eingeengt. Der Rückstand wird chromatographisch (Kieselgel, Essigester/Hexan 2:3) gereinigt.
   ¹H-NMR (500 Mhz, dmso): 1.15 (t, 3H), 1.32 (s, 9H), 3.2-3.4 (m, br, 9H), 4.05 (q, 2H), 4.3-4.4 (br, 2H), 7.53 (t, 1H), 7.64 (d, 1H), 7.73 (d, 1H), 7.75 (s, 1H).
   ¹³C-NMR: 14.9, 28.3 (3C), 40.7, 45.6 (2C), 53.7 (2C), 61.2, 89.0, 111.2, 119.2, 130.0, 131.5, 132.2, 133.6, 155.0, 157.6, 165.5. MS (ESI) : 432.5 [M⁺ + H], 376, 332, 185, 159.
1-Ethoxycarbonyl-4-[1-(3-cyanobenzyl)-1-hydrazino]-carbonylpiperazin XVI: 1 g XVII wird in 10 ml CH₂Cl₂ gelöst und bei 0 °C mit 3 ml Trifluoressigsäure versetzt. Das Reaktionsgemisch wird 3.5 Stunden bei Raumtemperatur gerührt und dann mit 10 %iger Zitronensäure extrahiert. Die wässrige Phase wird durch Zugabe von gesättigter Na₂CO₃-Lösung basisch gestellt und mit CH₂Cl₂ extrahiert. Die organische Phase wird eingeengt, und der Rückstand wird am Hochvakuum getrocknet. Ausbeute: 82%.
   ¹H-NMR (500 Mhz, dmso): 1.19 (t, 3H), 3.0-3.5 (m, br, 10H), 4.05 (q, 2H), 4.45 (s, 2H), 7.52 (t, 1H), 7.62 (d, 1H), 7.72 (s, 1H), 7.75 (d, 1H).
   ¹³C-NMR: 14.9, 40.7, 45.9 (2C), 56.0 (2C), 61.2, 111.4, 119.3, 129.6, 131.2, 132.4, 133.9, 155.0, 163.5, 169.1, 176.6. MS (ESI): 332.4 [M⁺ + H], 185, 159, 142, 101.
1-Ethoxycarbonyl-4-[1-(3-cyanobenzyl)-2-(2,4,6-triisopropylphenylsulfonyl)-1-hydrazino-carbonyl]-piperazin V: 1 mmol XVI wird in 4 ml CH₂Cl₂ gelöst und mit 1.5 mmol Triisopropylphenylsulfonylchlorid versetzt. Das Reaktionsgemisch wird zum Rückfluss erhitzt, mit 3.5 mmol DIPEA versetzt und 48 Stunden bei Rückfluss gerührt. Das Reaktionsgemisch wird mit 10 %iger Zitronensäure gewaschen und eingeengt. Der Rückstand wird chromatographisch (Kieselgel, Essigester/Hexan 1:3) gereinigt.
   ¹H-NMR (500 Mhz, dmso): 1.2-1.4 (m, 21H), 2.9 (m, 1H), 3.0-3.5 (m, 10H), 3.9 (m, 2H), 4.1 (q, 2H), 4.5 (br, 1H), 7.22 (s, 2H), 7.40-7.48 (m, 2H), 7.53 (s, 1H), 7.71 (dt, 1H). MS (ESI): 598.6 [M⁺ + H], 330, 251, 233, 185, 159.
1-Ethoxycarbonyl-4-[1-(3-(ethoxy-imino-methyl)-benzyl)-2-(2,4,6-triisopropylphenylsulfonyl)-1-hydrazino-carbonyl]-piperazin XXI: Salzsäure-Gas wird für 30 Minuten durch eine gekühlte Lösung von 1 mmol V in 30 ml Ethanol geleitet. Die Lösung wird für 1 h bei Raumtemperatur gerührt und anschliessend eingedampft. Ausbeute: 100%.
   MS (ESI): 644.6 [M⁺ + H], 481, 348, 163.
1-Ethoxycarbonyl-4-[1-(3-(amino-imino-methyl)-benzyl)-2-(2,4,6-triisopropylphenylsulfonyl)-1-hydrazino-carbonyl]-piperazin hydrochlorid VII: 1 mmol XXI wird in 15 ml Ethanol gelöst, mit 3 mmol NH₄OAc versetzt und 4 h bei 60 °C gerührt. Das Lösungsmittel wird abgedampft, und das Rohprodukt wird mittels präparativer HPLC (H₂O, Acetonitril) gereinigt. Ausbeute: 47%.
   ¹H-NMR (500 MHz, dmso): 1.1-1.3 (m, 21H), 2.91 (qq, 1H), 3.0-3.4 (m, 10H), 3.99 (qq, 2H), 4.04 (q, 2H), 7.20 (s, 2H), 7.30 (d, 1H), 7.48 (t, 1H), 7.63 (s, 1H), 7.70 (d, 1H), 9.18 (s, 1H), 9.38 (s, 1H), 9.56 (s, 1H).
   ¹³C-NMR: 28.5, 23.8 (4C), 25.5 (2C), 29.7, 33.8 (2C), 45.3, 56.4 (2C), 56.7 (2C), 61.2, 124.0 (2C), 126.0, 127.6, 128.0, 129.3, 133.0, 133.8, 137.3, 150.6 (2C), 155.4, 154.9, 160.8, 165.9.
   MS (ESI): 615.6 [M⁺ + H, freie Base].
1-Ethoxycarbonyl-4-[1-(3-(amino-hydroxyimino-methyl)-benzyl)-2-(2,4,6-triisopropylphenylsulfonyl)-1-hydrazino-carbonyl]-piperazin hydrochlorid VI: 1 mmol V wird in 30 ml Ethanol gelöst, mit 5 mmol Hydroxylamin-hydrochlorid und 2 ml 10 % NaCO₃ versetzt und 2 Stunden bei Rückfluss gerührt. Das Lösungsmittel wird abgedampft. Der Rückstand wird in 70 ml EtOAc gelöst und mit 60 ml Wasser und 50 ml 1N HCl gewaschen. Das Rohprodukt wird mittels präparativer HPLC (H₂O, Acetonitril) gereinigt.
   Ausbeute: 60%.
   ¹H-NMR (500 MHz, dmso): 1.2-1.4 (m, 21H), 2.9 (m, 1H), 3.0-3.5 (m, 10H), 3.9-4.1 (m, 4H), 4.4 (br, 1H), 7.05 (m, 3H), 7.28-7.35 (m, 1H), 7.53 (m, 2H), 9.3 (s, 1H).
   MS (ESI): 631.6 [M⁺ + H], 335, 150.

Nach den oben erwähnten Methoden können die in Tabelle 1 aufgelisteten Verbindungen hergestellt werden.

**Tabelle 1**

| Nr. | R¹ | R² | R³ |
|---|---|---|---|
| 1. | 4-Formylpiperazinyl | 2,4,6-Triisopropylphenyl | H |
| 2. | 4-Acetylpiperazinyl | 2,4,6-Triisopropylphenyl | H |
| 3. | 4-Propionylpiperazinyl | 2,4,6-Triisopropylphenyl | H |
| 4. | 4-Methoxycarbonylpiperazinyl | 2,4,6-Triisopropylphenyl | H |
| 5. | 4-Ethoxycarbonylpiperazinyl | 2,4,6-Triisopropylphenyl | H |
| 6. | 4-Propoxycarbonylpiperazinyl | 2,4,6-Triisopropylphenyl | H |
| 7. | 4-(n-Butoxy)-carbonylpiperazinyl | 2,4,6-Triisopropylphenyl | H |
| 8. | 4-Allyloxycarbonylpiperazinyl | 2,4,6-Triisopropylphenyl | H |
| 9. | 4-Propargyloxycarbonylpiperazinyl | 2,4,6-Triisopropylphenyl | H |
| 10. | 4-Aminocarbonylpiperazinyl | 2,4,6-Triisopropylphenyl | H |
| 11. | 4-Ethylaminocarbonylpiperazinyl | 2,4,6-Triisopropylphenyl | H |
| 12. | 4-Dimethylaminocarbonylpiperazinyl | 2,4,6-Triisopropylphenyl | H |
| 13. | 4-Diethylaminocarbonylpiperazinyl | 2,4,6-Triisopropylphenyl | H |
| 14. | 4-Benzyloxycarbonylpiperazinyl | 2,4,6-Triisopropylphenyl | H |
| 15. | 4-Ethoxycarbonylpiperazinyl | 2,4,6-Trimethylphenyl | H |
| 16. | 4-Ethoxycarbonylpiperazinyl | 2,4,6-Triisopropylphenyl | OH |
| 17. | 4-Acetylpiperazinyl | 2,4,6-Triisopropylphenyl | OH |
| 18. | 4-Ethoxycarbonylpiperazinyl | 2,4,6-Triisopropylphenyl | NH₂ |
| 19. | 4-Acetylpiperazinyl | 2,4,6-Triisopropylphenyl | NH₂ |

### Bestimmung der Urokinase-Hemmwirkung

Zur Bestimmung der Inhibitoraktivität werden 200 µl Tris-Puffer (0.05 mol/l, den Inhibitor enthaltend, 0.154 mol/l NaCl, 5% Ethanol, pH 8.0), 25 µl Substrat (Pefachrome UK oder Bz-ßAla-Gly-Arg-pNA in H₂O; Pentapharm Ltd., Basel, Schweiz) und 50 µl sc-Urokinase (Ribosepharm GmbH, Haan, Deutschland) bei 25°C inkubiert. Nach 3 min wird die Reaktion durch Zugabe von 25 µl Essigsäure (50%) unterbrochen und die Absorption bei 405 nm mittels Microplate Reader (MR 50001 Dynatech, Denkendorf, Deutschland) bestimmt. Die Kᵢ-Werte werden nach Dixon durch lineare Regression mittels eines Computerprogramms ermittelt. Die Kᵢ-Werte sind das Mittel aus mindestens 3 Bestimmungen.

Nach der oben erwähnten Methode können die in Tabelle 2 erwähnten Kᵢ-Werte ermittelt werden.

**Tabelle 2**

| Verbindung von Beispiel | Kᵢ in µMol/l |
|---|---|
| 5 | 0.67 ± 0.18 |
| 18 | 25.6 ± 9.5 |

## Patentansprüche

1. Verbindungen der allgemeinen Formel I 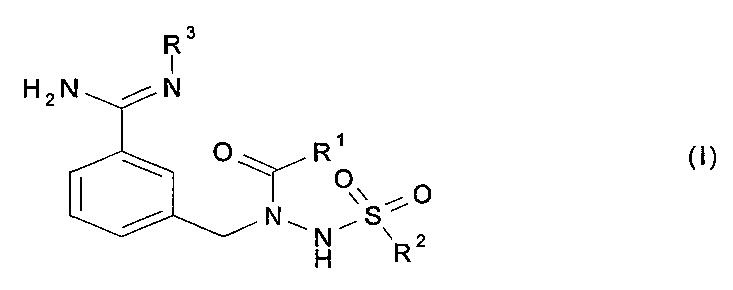 die bezüglich R¹ als Racemate sowie als L- bzw. D-konfigurierte Verbindungen und als E/Z-Gemische sowie als E- bzw. Z-Isomere vorliegen und.in denen
R¹
(a) OH oder gegebenenfalls mit Aryl substituiertes (C₁-C₈)-Alkoxy oder (C₃-C₈)-Cycloalkyloxy bedeutet; oder
(b) eine Gruppe der Formel -N(R⁴R⁵) bedeutet, in welcher R⁴ und R⁵ unabhängig voneinander Wasserstoff, (C₁-C₈)-Alkyl, mit gegebenenfalls 1- bis 3-fach identisch oder unterschiedlich substituiertem Aryl substituiertes (C₁-C₈)-Alkyl, (C₃-C₈)-Cycloalkyl, (C₃-C₈)-Cycloalkyl-(C₁-C₄)-alkyl, NH₂, NHR⁶ oder NR⁶R⁷ bedeuten, wobei R⁶ und R⁷ unabhängig voneinander (C₁-C₈)-Alkyl, (C₃-C₈)-Cycloalkyl, gegebenenfalls 1- bis 3-fach identisch oder unterschiedlich substituiertes Aryl, gegebenenfalls 1-bis 3-fach identisch oder unterschiedlich substituiertes Heteroaryl bedeuten, oder R⁶ und R⁷ zusammen mit dem Stickstoffatom, an das sie gebunden sind, einen Thiazolin-, Thiazolidin-, Oxazolin-, Oxazolidin- oder Morpholinring bedeuten, welcher gegebenenfalls 1- bis 3-fach identisch oder unterschiedlich substituiert sein kann; oder
(c) eine Gruppe der Formel 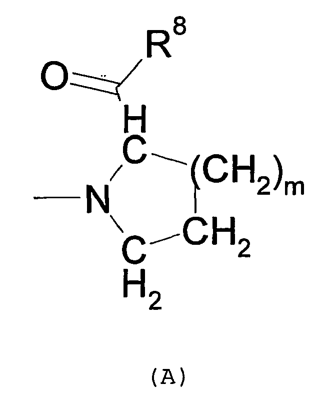 bedeutet, in welcher m die Zahl 1 oder 2 bedeutet und in welcher eine der Methylengruppen mit einem Hydroxyl-, Carboxyl- oder (C₁-C₈)-Alkylrest oder einem mit gegebenenfalls 1- bis 3-fach identisch oder unterschiedlich substituiertem Aryl substituierten (C₁-C₈)-Alkylrest substituiert sein kann und R⁸ eine der oben unter (a) und (b) angegebenen Bedeutungen von R¹ hat, wobei in 2,3- oder 3,4-Stellung, bezogen auf das Stickstoffatom, gegebenenfalls ein weiterer aromatischer oder cycloaliphatischer Ring ankondensiert ist; oder
(d) eine Gruppe der Formel 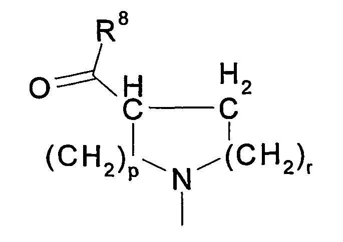 bedeutet, in welcher p und r unabhängig voneinander 1 oder 2 sind und in welcher eine der Methylengruppen mit einem Hydroxyl-, Carboxyl-, (C₁-C₈)-Alkyl- oder einem mit gegebenenfalls 1- bis 3-fach identisch oder unterschiedlich substituiertem Aryl substituierten (C₁-C₈)-Alkylrest substituiert sein kann und R⁸ eine der oben unter (a) und (b) angegebenen Bedeutungen von R¹ hat, wobei in 2,3- oder 3,4-Stellung, bezogen auf das Stickstoffatom, gegebenenfalls ein weiterer aromatischer oder cycloaliphatischer Ring ankondensiert ist; oder
(e) eine Piperidylgruppe bedeutet, die gegebenenfalls in einer der Stellungen 2, 3 und 4 mit einem (C₁-C₈)-Alkylcarbonyl-, einem (C₁-C₈)-Alkoxycarbonyl-, einem (C₁-C₄)-Alkyl- oder einem Hydroxylrest substituiert ist, wobei in 2,3- oder 3,4-Stellung, bezogen auf das Stickstoffatom, gegebenenfalls ein weiterer aromatischer oder cycloaliphatischer Ring ankondensiert ist; oder
(f) eine Gruppe der Formel 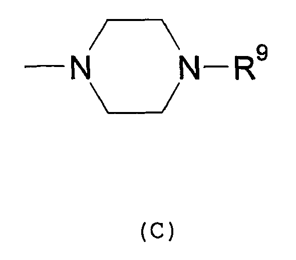 bedeutet, worin R⁹ H, (C₁-C₈)-Alkyl, (C₂-C₈)-Alkenyl, (C₂-C₈)-Alkinyl, Formyl, (C₁-C₈)-Alkylcarbonyl,C₂-C₈)-Alkenylcarbonyl, (C₂-C₈)-Alkinylcarbonyl, Cyano, (C₁-C₈)-Alkoxycarbonyl, (C₂-C₈)-Alkenyloxycarbonyl, (C₂-C₈)-Alkinyloxycarbonyl, (C₃-C₈) -Cycloalkyl, (C₃-C₈) Cycloalkylcarbonyl, (C₃-C₈)-Cycloalkyloxycarbonyl; (C₃-C₈)-Heterocyclyl, (C₃-C₈) Heterocyclylcarbonyl, (C₃-C₈)-Heterocyclyloxycarbonyl mit jeweils 1 bis 3 Heteroatomen; Aryl, Arylcarbonyl, Aryloxycarbonyl; Heteroaryl, Heteroarylcarbonyl, Heteroaryloxycarbonyl, mit jeweils 1 bis 3 Heteroatomen; einen Carbonsäureamidrest der Formel -CON(R⁴R⁵) oder einen Thiocarbonsäureamidrest der Formel -CSN(R⁴R⁵) bedeutet, in welchen R⁴ und R⁵ unabhängig voneinander Wasserstoff, (C₁-C₈)-Alkyl, (C₂-C₈)-Alkenyl, (C₂-C₈)-Alkinyl, (C₃-C₈)-Cycloalkyl, Aryl, oder Heteroaryl bedeuten oder R⁴ und R⁵ zusammen mit dem Stickstoffatom, an das sie gebunden sind, einen 5- bis 7-gliedrigen Ring bedeuten, der 1 bis 2 weitere Heteroatome (N, O, S) enthalten kann und der gegebenenfalls 1- bis 3-fach identisch oder unterschiedlich substituiert sein kann oder einen -SO₂Y-Rest bedeutet, in welchem Y (C₁-C₈)-Alkyl, (C₂-C₈)-Alkenyl, (C₂-C₈)-Alkinyl, (C₃-C₈)-Cycloalkyl, Aryl, Heteroaryl, Aryloxy oder N(R⁴R⁵) bedeutet; wobei alle Alkyl- Alkenyl- und Alkinylreste der Alkyl, Alkenyl bzw Alkinyl enthaltenden Gruppen 1-bis 3-fach identisch oder unterschiedlich substituiert und alle Cycloalkyl-, Heterocyclyl-, Aryl- und Heteroarylreste der Cycloalkyl, Heterocyclyl, Aryl bzw Heteroaryl enthaltenden Gruppen 1- bis 3-fach identisch oder unterschiedlich substituiert sein können;
R² einen gegebenenfalls 1- bis 3-fach identisch oder unterschiedlich substituierten Aryl- oder Heteroarylrest oder einen Campherrest bedeutet; und
R³ Wasserstoff, Amino oder Hydroxy bedeutet;
wobei diese Verbindungen sowohl als freie Basen als auch als Salze mit Mineralsäuren oder als Salze mit organischen Säuren vorliegen können.

2. Verbindungen gemäss Anspruch 1, worin R¹ OH, gegebenenfalls mit Aryl substituiertes (C₁-C₈)-Alkoxy, (C₃-C₈)-Cycloalkyloxy oder eine Gruppe der Formel -NR⁴R⁵, (B) oder (C) bedeutet.

3. Verbindungen gemäss Anspruch 2, worin R¹ eine Gruppe der Formel (C) bedeutet, worin R⁹ Formyl, (C₁-C₈)-Alkylcarbonyl, (C₂-C₈)-Alkenylcarbonyl, (C₂-C₈)-Alkinylcarbonyl, (C₁-C₈)-Alkoxycarbonyl, (C₂-C₈)-Alkenyloxycarbonyl, (C₂-C₈)-Alkinyloxycarbonyl oder einen Carbonsäureamidrest der Formel -CON(R⁴R⁵) bedeutet, wobei R⁴ und R⁵ je Wasserstoff, (C₁-C₈)-Alkyl, (C₂-C₈)-Alkenyl oder (C₂-C₈)-Alkinyl bedeuten.

4. Verbindungen gemäss Anspruch 3, worin R⁹ Methoxycarbonyl, Ethoxycarbonyl, Benzyloxycarbonyl, Dimethylaminocarbonyl oder Acetyl bedeutet.

5. Verbindungen gemäss einem der Ansprüche 1 bis 4, worin R² Phenyl, 4-Tolyl, 2,4,6-Trimethylphenyl, 2,4,6-Triisopropylphenyl, 4-Methoxy-2,3,6-trimethylphenyl, 2,2-Dimethyl-6-methoxychromanyl, 2,2,5,7,8-Pentamethylchromanyl, Anthrachinonyl, 1-Naphthyl, 2-Naphthyl, 5-(Dimethylamino)-naphthyl, Chinolyl, Isochinolyl, oder einen Campherrest bedeutet.

6. Verbindungen gemäss Anspruch 5, worin R² 2,4,6-Triisopropylphenyl bedeutet.

7. Verbindungen gemäss einem der Ansprüche 1 bis 6, worin R³ Wasserstoff bedeutet.

8. Verbindungen gemäss einem der Ansprüche 1 bis 7, **dadurch gekennzeichnet, dass** sie in Form von physiologisch verträglichen Säuresalzen vorliegen.

9. Verbindungen gemäss einem der Ansprüche 1 bis 8 zur Anwendung als therapeutische Wirkstoffe oder für diagnostische Zecke.

10. Arzneimittel, enthaltend eine Verbindung gemäss einem der Ansprüche 1 bis 8 oder ein Salz davon und gegebenenfalls mindestens einen für ein Arzneimittel geeigneten Hilfsstoff.

11. Diagnostisches Mittel, enthaltend eine Verbindung gemäss einem der Ansprüche 1 bis 8 oder ein Salz davon und gegebenenfalls mindestens einen für ein diagnostisches Mittel geeigneten Hilfsstoff.

12. Verwendung der Verbindungen nach einem der Ansprüche 1 bis 8 zur Herstellung eines Mittels für die Diagnostik, Therapie und Prävention von Urokinase- oder Urokinaserezeptor-assoziierten Krankheiten.

13. Verwendung nach Anspruch 12 zur Herstellung eines Mittels für die Tumorbekämpfung.

14. Verwendung nach Anspruch 13 zur Herstellung eines Mittels für die Bekämpfung von Mammakarzinomen, Pankreaskarzinomen und der Metastasenbildung.

15. Verwendung nach Anspruch 12 zur Herstellung eines Mittels für die Bekämpfung von Pemphigus vulgaris.

16. Mittel bzw. Verwendung nach einem der Ansprüche 10 bis 15, **dadurch gekennzeichnet, dass** man eine oder mehrere der Verbindungen gemäss einem der Ansprüche 1 bis 8 in Kombination mit mindestens einer weiteren pharmakologisch wirksamen Substanz einsetzt.

17. Mittel bzw. Verwendung nach Anspruch 16, **dadurch gekennzeichnet, dass** man eine oder mehrere der Verbindungen gemäss einem der Ansprüche 1 bis 8 in Kombination mit mindestens einer Radiomarkierung und/oder mindestens einer zytotoxischen Substanz einsetzt.

18. Mittel bzw. Verwendung nach einem der Ansprüche 10 und 12 bis 17, **dadurch gekennzeichnet, dass** es sich um ein oral, topisch, rektal oder parenteral verabreichbares Arzneimittel handelt.

19. Mittel bzw. Verwendung nach Anspruch 18, **dadurch gekennzeichnet, dass** das Arzneimittel in Form von Tabletten, Dragees, Kapseln, Pellets, Suppositorien, Lösungen, Pflastern oder anderen transdermalen Systemen vorliegt.

20. Verfahren zur Herstellung von Verbindungen der in Anspruch 1 definierten Formel I und von Säureadditionssalzen davon, **dadurch gekennzeichnet, dass** man
(a) zur Herstellung einer Verbindung der Formel I, worin R³ Wasserstoff oder Amino bedeutet, eine Cyanoverbindung der Formel 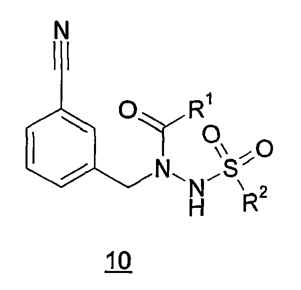 worin R¹ und R² die in Anspruch 1 angegebene Bedeutung besitzen, durch Addition von H₂S an die Cyangruppe in das entsprechende Thioamid überführt, aus welcher durch S-Alkylierung mit ein Thioimidoester erhalten wir, welcher anschliessend durch Behandlung mit Ammoniak oder Hydrazin oder einem Salz davon in die Amidino- bzw. Amidrazonverbindung übergeführt wird; oder
(b) zur Herstellung einer Verbindung der Formel I, worin R³ Wasserstoff oder Amino bedeutet, eine Cyanoverbindung der Formel 10 durch saure Alkoholyse in ein Säureadditionssalz des entsprechenden Imidoesters überführt, dessen Umsetzung mit Ammoniak oder Hydrazin zur Amidino- bzw. Amidrazonverbindung führt; oder
(c) zur Herstellung einer Verbindung der Formel I, worin R³ Wasserstoff bedeutet, eine Verbindung der Formel I, worin R³ OH bedeutet, oder eine entsprechende Alkanoyloxyverbindung, reduziert; oder
(d) zur Herstellung einer Verbindung der Formel I, worin R³ OH oder NH₂ bedeutet, eine Cyanoverbindung der Formel 10 mittels Hydroxylamin oder Hydrazin in die entsprechende Hydroxyamidin- bzw. Amidrazonverbindung überführt; und
(e) erwünschtenfalls eine erhaltene Verbindung der Formel I in ein Säureadditionssalz überführt und/oder ein erhaltenes Säureadditionssalz in die entsprechende Verbindung der Formel I oder in ein anderes Säureadditionssalz überführt.

21. Verbindungen der in Anspruch 20 definierten Formel 10.

## Claims

1. Compounds of the general formula I 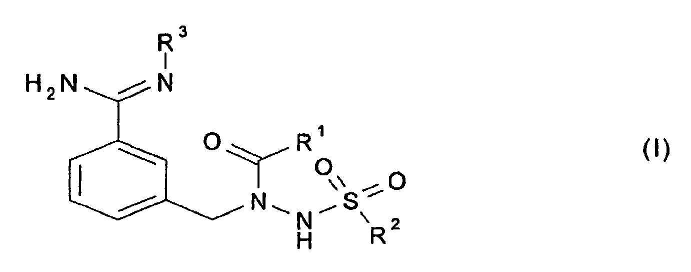 which with reference to R¹ are present as racemates as well as L- or D-configured compounds and as E/Z mixtures as well as E- or Z-isomers, and in which
R¹ denotes
(a) OH or (C₁-C₈) alkoxy optionally substituted with aryl or (C₃-C₈) cycloalkyloxy; or
(b) a group of formula -N(R⁴R⁵) in which R⁴ and R⁵ independently of each other represent hydrogen, (C₁-C₈) alkyl, (C₁-C₈) alkyl optionally substituted with aryl which is optionally identically or differently substituted once to three times, (C₃-C₈) cycloalkyl, (C₃-C₈)-cycloalkyl-(C₁-C₄)-alkyl, NH₂, NHR⁶ or NR⁶R⁷ in which R⁶ and R⁷ independently of each other represent (C₁-C₈) alkyl, (C₃-C₈) cycloalkyl, aryl which is optionally identically or differently substituted once to three times, heteroaryl which is optionally identically or differently substituted once to three times or R⁶ and R⁷ together with the nitrogen atom to which they are bound represent a thiazoline, thiazolidine, oxazoline, oxazolidine or morpholine ring, which can be optionally identically or differently substituted once to three times; or
(c) a group of formula 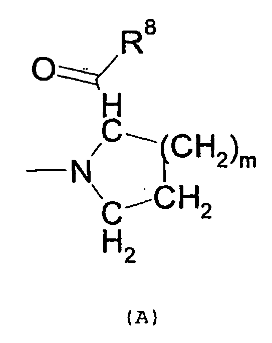 in which m denotes the number 1 or 2 and in which one of the methylene groups can be substituted with a hydroxyl, carboxyl or (C₁-C₈) alkyl residue or a (C₁-C₈) alkyl residue substituted with aryl which is optionally identically or differently substituted once to three times and R⁸ has one of the above meanings for R¹ stated under (a) and (b), wherein a further aromatic or cycloaliphatic ring is optionally condensed in the 2,3 or 3,4 position relative to the nitrogen atom; or
(d) a group of formula 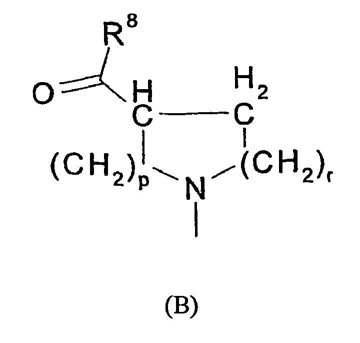 in which p and r independently of each other are 1 or 2 and in which one of the methylene groups can be substituted with a hydroxyl, carboxyl, (C₁-C₈) alkyl or a (C₁-C₈) alkyl residue substituted with aryl which is optionally identically or differently substituted once to three times and R⁸ has one of the above meanings for R¹ stated under (a) and (b), wherein a further aromatic or cycloaliphatic ring is optionally condensed in the 2,3 or 3,4 position relative to the nitrogen atom; or
(e) a piperidyl group which is optionally substituted with a (C₁-C₈) alkylcarbonyl, a (C₁-C₈) alkoxycarbonyl, a (C₁-C₄) alkyl or a hydroxyl residue in one of the positions 2, 3 and 4, wherein a further aromatic or cycloaliphatic ring is optionally condensed in the 2,3 or 3,4 position relative to the nitrogen atom; or
(f) a group of formula 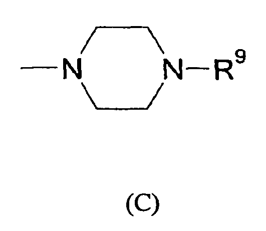 in which R⁹ represents H, (C₁-C₈) alkyl, (C₂-C₈) alkenyl, (C₂-C₈) alkinyl, formyl, (C₁-C₈) alkylcarbonyl, (C₂-C₈) alkenylcarbonyl, (C₂-C₈) alkinylcarbonyl, cyano, (C₁-C₈) alkoxycarbonyl, (C₂-C₈) alkenyloxycarbonyl, (C₂-C₈) alkinyloxycarbonyl, (C₃-C₈) cycloalkyl, (C₃-C₈) cycloalkylcarbonyl, (C₃-C₈) cycloalkyloxycarbonyl, (C₃-C₈) heterocyclyl, (C₃-C₈) heterocyclylcarbonyl, (C₃-C₈) heterocyclyloxycarbonyl with 1 to 3 heteroatoms each; aryl, arylcarbonyl, aryloxycarbonyl; heteroaryl, heteroarylcarbonyl, heteroaryloxycarbonyl with 1 to 3 heteroatoms each; a carboxamide residue of formula -CON(R⁴-R⁵) or a thiocarboxamide residue of formula -CSN(R⁴R⁵), wherein R⁴ and R⁵ independently of each other represent hydrogen, (C₁-C₈) alkyl, (C₂-C₈) alkenyl, (C₂-C₈) alkinyl, (C₃-C₈) cycloalkyl, aryl or heteroaryl or R⁴ and R⁵ together with the nitrogen atom to which they are bound denote a 5- to 7-membered ring which can comprise 1 to 2 further heteroatoms (N, O, S) and which can optionally be identically or differently substituted once to three times or an -SO₂Y residue, wherein Y represents (C₁-C₈) alkyl, (C₂-C₈) alkenyl, (C₂-C₈) alkinyl, (C₃-C₈) cycloaryl, aryl, heteroaryl, aryloxy or N(R⁴R⁵); wherein all the alkyl, alkenyl and alkinyl residues of the groups containing alkyl, alkenyl and alkinyl can be identically or differently substituted once to three times and all the cycloalkyl, heterocyclyl, aryl, and heteroaryl residues of the groups containing cycloalkyl, heterocyclyl, aryl, and heteroaryl can be identically or differently substituted once to three times;
R² represents an aryl or heteroaryl residue optionally identically or differently substituted once to three times or a camphor residue; and
R³ represents hydrogen, amino or hydroxy;
wherein these compounds can be present as free bases as well as salts with inorganic acids or as salts with organic acids.

2. Compounds according to claim 1, wherein R¹ denotes OH, (C₁-C₈) alkoxy optionally substituted with aryl, (C₃-C₈) cycloalkyloxy or a group of formula -NR⁴R⁵, (B) or (C).

3. Compounds according to claim 2, wherein R¹ denotes a group of formula (C) in which R⁹ denotes formyl, (C₁-C₈) alkylcarbonyl, (C₂-C₈) alkenylcarbonyl, (C₂-C₈) alkinylcarbonyl, (C₁-C₈) alkoxycarbonyl, (C₂-C₈) alkenyloxycarbonyl, (C₂-C₈) alkinyloxycarbonyl or a carboxamide residue of the formula
- CON(R⁴R⁵) in which R⁴ and R⁵ each denote hydrogen, (C₁-C₈) alkyl, (C₂-C₈) alkenyl or (C₂-C₈) alkinyl.

4. Compounds according to claim 3, wherein R⁹ denotes methoxycarbonyl, ethoxycarbonyl, benzyloxycarbonyl, dimethylamino-carbonyl or acetyl.

5. Compounds according to one of the claims 1 to 4, wherein R² denotes phenyl, 4-tolyl, 2,4,6-trimethylphenyl, 2,4,6-triisopropylphenyl, 4-methoxy-2,3,6-trimethylphenyl, 2,2-dimethyl-6-methoxychromanyl, 2,2,5,7,8-pentamethylchromanyl, anthraquinonyl, 1-naphthyl, 2-naphthyl, 5-(dimethylamino)-naphthyl, quinolyl, isoquinolyl or a camphor residue.

6. Compounds according to claim 5, wherein R² denotes 2,4,6-triisopropylphenyl.

7. Compounds according to one of the claims 1 to 6, wherein R³ denotes hydrogen.

8. Compounds according to one of the claims 1 to 7, **characterized in that** they are present in the form of physiologically compatible acid salts.

9. Compounds according to one of the claims 1 to 8 for use as therapeutic agents or for diagnostic purposes.

10. Pharmaceutical preparation containing a compound according to one of the claims 1 to 8 or a salt thereof and optionally at least one auxiliary agent suitable for a pharmaceutical preparation.

11. Diagnostic agent containing a compound according to one of the claims 1 to 8 or a salt thereof and optionally at least one auxiliary agent suitable for a diagnostic agent.

12. Use of the compounds according to one of the claims 1 to 8 for the production of an agent for the diagnosis, therapy and prevention of urokinase-associated or urokinase receptor-associated diseases.

13. Use according to claim 12 for the production of an antitumoral agent.

14. Use according to claim 13 for the production of an agent against mamma carcinomas, pancreatic carcinomas and formation of metastases.

15. Use according to claim 12 for the production of an agent against Pemphigus vulgaris.

16. Agent or use according to one of the claims 10 to 15, **characterized in that** one or more of the compounds according to one of the claims 1 to 8 is used in combination with at least one further pharmacologically active substance.

17. Agent or use according to claim 16, **characterized in that** one or more of the compounds according to one of the claims 1 to 8 are used in combination with at least one radiolabel and/or at least one cytotoxic substance.

18. Agent or use according to one of the claims 10 and 12 to 17, **characterized in that** it is a pharmaceutical preparation that can be administered orally, topically, rectally or parenterally.

19. Agent or use according to claim 18, **characterized in that** the pharmaceutical preparation is present in the form of tablets, dragées, capsules, pellets, suppositories, solutions, plasters or other transdermal systems.

20. Process for the production of the compounds of formula I defined in claim 1 and of acid addition salts thereof, **characterized in that**
(a) for the synthesis of a compound of formula I in which R³ denotes hydrogen or amino, a cyano compound of formula 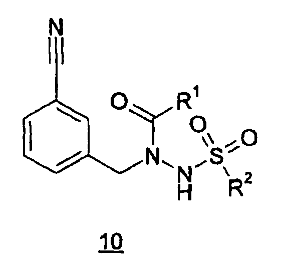 wherein R¹ and R² have the meaning given in claim 1 is converted by addition of H₂S to the cyano group into the corresponding thioamide from which a thioimido ester is obtained by S-alkylation which is subsequently converted into the amidino or amidrazone compound, respectively by treatment with ammonia or hydrazine or a salt thereof; or
(b) for the synthesis of a compound of formula I in which R³ denotes hydrogen or amino, a cyano compound of formula 10 is converted by acid alcoholysis into an acid addition salt of the corresponding imido ester, the reaction of which with ammonia or hydrazine forms the amidino or amidrazone compound respectively; or
(c) for the synthesis of a compound of formula I in which R³ denotes hydrogen, a compound of formula I in which R³ denotes OH or a corresponding alkanoyloxy compound is reduced; or
(d) for the synthesis of a compound of formula I in which R³ denotes OH or NH₂, a cyano compound of formula 10 is converted into the corresponding hydroxyamidine or amidrazone compound respectively, by means of hydroxylamine or hydrazine and
(e) if desired, a compound obtained of formula I is converted into an acid addition salt and/or an acid addition salt obtained is converted into the corresponding compound of formula I or into another acid addition salt.

21. Compounds of formula 10 defined in claim 20.

## Revendications

1. Composés de formule générale I 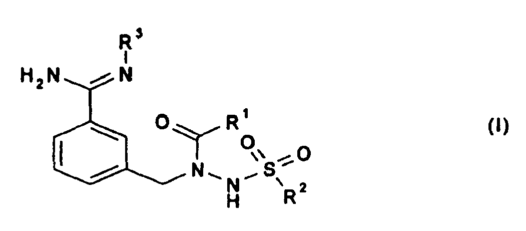 qui, relativement à R¹, existent sous forme de racémates, ainsi que de composés de configuration L et/ou D et de mélanges E/Z ainsi que d'isomères E et/ou Z, et où
R¹ (a) représente OH ou un alcoxy-(C₁-C₈) substitué le cas échéant par un aryle ou un cycloalkyloxy-(C₃-C₈) ; ou (b) représente un groupe de formule -N(R⁴R⁵), où R⁴ et R⁵ sont, indépendamment l'un de l'autre, un atome d'hydrogène, un alkyle-(C₁-C₈), un alkyle-(C₁-C₈) substitué le cas échéant par un aryle substitué de 1 à 3 fois de manière identique ou différente, un cycloalkyle-(C₃-C₈), un cycloalkyle-(C₃-C₈)-alkyle-(C₁-C₄), NH₂, NHR⁶ ou NR⁶R⁷, R⁶ et R⁷ étant, indépendamment l'un de l'autre, un alkyle-(C₁-C₈), un cycloalkyle-(C₃-C₈), un aryle substitué le cas échéant de 1 à 3 fois de manière identique ou différente, un hétéroaryle substitué le cas échéant de 1 à 3 fois de manière identique ou différente, ou R⁶ et R⁷ étant, conjointement par l'atome d'azote auquel ils sont liés, un cycle thiazoline, thiazolidine, oxazoline, oxazolidine ou morpholine, lequel peut être substitué le cas échéant de 1 à 3 fois de manière identique ou différente ; ou (c) représente un groupe de formule 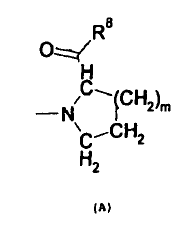 où m représente le nombre 1 ou 2 et où un des groupes méthylène peut être substitué par un résidu hydroxyle, carboxyle ou alkyle-(C₁-C₈) ou par un alkyle-(C₁-C₈) substitué le cas échéant de 1 à 3 fois de manière identique ou différente par un aryle et R⁸ a une des significations données en (a) et (b) pour R¹, où un autre cycle aromatique ou cycloaliphatique est le cas échéant condensé en position 2,3- ou 3,4-, relativement à l'atome d'azote ; ou (d) représente un groupe de formule 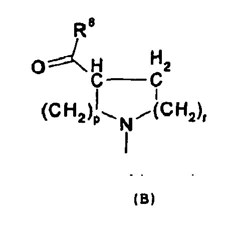 où p et r sont, indépendamment l'un de l'autre, 1 ou 2 et où un des groupes méthylène peut être substitué par un résidu hydroxyle, carboxyle ou alkyle-(C₁-C₈) ou par un alkyle-(C₁-C₈) substitué le cas échéant de 1 à 3 fois de manière identique ou différente par un aryle et R⁸ a une des significations données en (a) et (b) pour R¹, dans lesquelles un autre cycle aromatique ou cycloaliphatique est le cas échéant condensé en position 2,3- ou 3,4-, relativement à l'atome d'azote ; ou (e) représente un groupe piperidyle qui est substitué le cas échéant en l'une des positions 2, 3 et 4 par un résidu alkylcarbonyle-(C₁-C₈), un résidu alcoxycarbonyle-(C₁-C₈), un résidu alkyle-(C₁-C₄) ou un résidu hydroxyle, où un autre cycle aromatique ou cycloaliphatique est le cas échéant condensé en position 2,3- ou 3,4-, relativement à l'atome d'azote ; ou (f) représente un groupe de formule 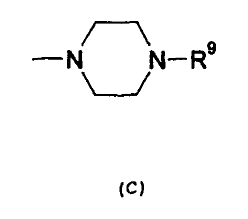 où R⁹ représente H, un alkyle-(C₁-C₈), un alcényle-(C₂-C₈), un alcinyle-(C₂-C₈), un formyle, un alkylcarbonyle-(C₁-C₈), un alcénylcarbonyle-(C₂-C₈), un alcinylcarbonyle-(C₂-C₈), un groupe cyano, un alcoxycarbonyle-(C₁-C₈), un alcényloxycarbonyle-(C₂-C₈), un alcinyloxycarbonyle-(C₂-C₈), un cycloalkyle-(C₃-C₈), un cycloalkylcarbonyle-(C₃-C₈), un cydoalkyloxycarbonyle-(C₃-C₈), un hétérocyclyle-(C₃-C₈), un hétérocyclylcarbonyle-(C₃-C₈), un hétérocyclyloxycarbonyle-(C₃-C₈) avec respectivement de 1 à 3 hétéroatomes ; un aryle, un arylcarbonyle, un aryloxycarbonyle ; un hétéroaryle, un hétéroarylcarbonyle, un hétéroaryloxycarbonyle avec respectivement de 1 à 3 hétéroatomes ; un résidu d'amide d'acide carboxylique de formule -CON(R⁴R⁵) ou un résidu d'amide d'acide thiocarboxylique de formule -CSN(R⁴R⁵), où R⁴ et R⁵ représentent, indépendamment l'un de l'autre, un atome d'hydrogène, un alkyle-(C₁-C₈), un alcényle-(C₂-C₈), un alcinyle-(C₂-C₈), un cycloalkyle-(C₃-C₈), un aryle ou un hétéroaryle, ou bien R⁴ et R⁵, conjointement par l'atome d'azote auquel ils sont liés, représentent un cycle de 5 à 7 chaînons, lequel peut contenir de 1 à 2 autres hétéroatomes (N, O, S) et être substitué le cas échéant de 1 à 3 fois de manière identique ou différente, ou représente un résidu -SO₂Y, où Y représente un alkyle-(C₁-C₈), un alcényle-(C₂-C₈), un alcinyle-(C₂-C₈), un cycloalkyle-(C₃-C₈), un aryle, un hétéroaryle, un aryloxy ou N(R⁴R⁵); où tous les résidus alkyle, alcényle et alcinyle des groupes contenant un alkyle, un alcényle et/ou un alcinyle peuvent être substitués de 1 à 3 fois de manière identique ou différente et où tous les résidus cycloalkyle, hétérocyclyle, aryle et hétéroaryle des groupes contenant un cycloalkyle, un hétérocyclyle, un aryle et/ou un hétéroaryle peuvent être substitués de 1 à 3 fois de manière identique ou différente ;
R² représente un résidu aryle substitué le cas échéant de 1 à 3 fois de manière identique ou différente ou un résidu hétéroaryle ou un résidu camphre ;
R³ représente un atome d'hydrogène, un groupe amino ou hydroxyle ;
ces composés peuvent exister aussi bien en tant que base libre qu'en tant que sels par des acides minéraux ou en tant que sels par des acides organiques.

2. Composés selon la revendication 1, où R¹ représente OH, un alkoxy-(C₁-C₈) substitué le cas échéant par un aryle, un cycloalkyloxy-(C₃-C₈) ou un groupe de formule -NR⁴R⁵, (B) ou (C).

3. Composés selon la revendication 2, où R¹ représente un groupe de formule (C), où R⁹ représente un formyle, alkylcarbonyle-(C₁-C₈), alcénylcarbonyle-(C₂-C₈), alcinylcarbonyle-(C₂-C₈), alkoxycarbonyle-(C₁-C₈), alcényloxycarbonyle-(C₂-C₈), alcinyloxycarbonyle-(C₂-C₈) ou un résidu d'amide d'acide carboxylique de formule -CON(R⁴R⁵), R⁴ et R⁵ représentant chacun un atome d'hydrogène, un alkyle-(C₁-C₈), un alcényle-(C₂-C₈) ou un alcinyle-(C₂-C₈).

4. Composés selon la revendication 3, où R⁹ représente du méthoxycarbonyle, de l'éthoxycarbonyle, du benzyloxycarbonyle, du diméthylaminocarbonyle ou de l'acétyle.

5. Composés selon l'une quelconque des revendications 1 à 4, où R² représente du phényle, du 4-tolyle, du 2,4,6-triméthylphényle, du 2,4,6-triisopropylphényle, du 4-méthoxy-2,3,6-triméthylphényle, du 2,2-diméthyl-6-méthoxychromanyle, du 2,2,5,7,8-pentaméthylchromanyle, de l'anthraquinonyle, du 1-napthyle, du 2-napthyle, du 5-(diméthylamino)-naphtyle, du quinolyle, de l'isoquinolyle ou un résidu camphre.

6. Composés selon la revendication 5, où R² représente du 2,4,6-triisopropylphényle.

7. Composés selon l'une quelconque des revendications 1 à 6, où R³ représente un atome d'hydrogène.

8. Composés selon l'une quelconque des revendications 1 à 7, **caractérisés en ce qu'**ils existent sous la forme de sels d'acides physiologiquement compatibles.

9. Composés selon l'une quelconque des revendications 1 à 8 pour utilisation en tant qu'agent thérapeutique ou à des fins de diagnostic.

10. Médicament contenant un composé selon l'une quelconque des revendications 1 à 8 ou un sel de ce composé et le cas échéant au moins un des adjuvants approprié pour un médicament.

11. Agent diagnostic contenant un composé selon l'une quelconque des revendications 1 à 8 ou un sel de ce composé et le cas échéant au moins un des adjuvants approprié pour un agent diagnostic.

12. Utilisation des composés selon l'une quelconque des revendications 1 à 8 pour la fabrication d'un agent pour le diagnostic, la thérapie et la prévention des maladies associées à l'urokinase ou aux récepteurs de l'urokinase.

13. Utilisation selon la revendication 12 pour la fabrication d'un agent de lutte contre les tumeurs.

14. Utilisation selon la revendication 13 pour la fabrication d'un agent de lutte contre le cancer du sein, le cancer du pancréas et la formation de métastases.

15. Utilisation selon la revendication 12 pour la fabrication d'un agent de lutte contre le pemphigus vulgaire.

16. Agent et/ou utilisation selon l'une quelconque des revendications 10 à 15, **caractérisé**(e) en ce que l'on utilise un ou plusieurs des composés selon l'une des revendications 1 à 8 en combinaison avec au moins une autre substance pharmacologiquement active.

17. Agent et/ou utilisation selon la revendication 16, **caractérisé**(e) en ce que l'on utilise un ou plusieurs des composés selon l'une quelconque des revendications 1 à 8 en combinaison avec au moins une substance de radiomarquage et/ou au moins une substance cytotoxique.

18. Agent et/ou utilisation selon l'une quelconque des revendications 10 et 12 à 17, **caractérisé**(e) en ce qu'il s'agit d'un médicament administrable par voie orale, topique, rectale ou parentérale.

19. Agent et/ou utilisation selon la revendication 18, **caractérisé**(e) en ce que le médicament existe sous forme de comprimés, dragées, capsules, granulés, suppositoires, solutions, pansements ou autres systèmes transdermiques.

20. Procédé pour la fabrication des composés définis par la formule I de la revendication 1 et des sels d'addition d'acide de ce composé, **caractérisé**(e) en ce que
(a) pour la fabrication d'un composé de formule I, où R³ représente un atome d'hydrogène ou un groupe amino, on transforme un composé cyano de formule 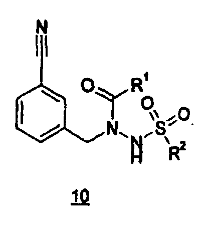 où R¹ et R² ont la signification donnée dans la revendication 1, par addition de H₂S sur le groupe cyano, conduisant au thioamide correspondant, à partir duquel on obtient, par S-alkylation, un thioimidoester, lequel est transformé ensuite, par traitement par de l'ammoniac ou de l'hydrazine ou un sel de ce composé, en le composé amidino et/ou amidrazone ; ou
(b) pour la fabrication d'un composé de formule I, où R³ représente un atome d'hydrogène ou un groupe amino, on transforme un composé cyano de formule 10 par alcoolyse acide en un sel d'addition acide de l'imidoester correspondant, dont la réaction avec de l'ammoniac ou de l'hydrazine conduit à la formation d'un composé amidino et/ou amidrazone ; ou
(c) pour la fabrication d'un composé de formule I, où R³ représente un atome d'hydrogène, on réduit un composé de formule I, où R³ représente OH, ou un composé alkanoyloxy correspondant ; ou
(d) pour la fabrication d'un composé de formule I, où R³ représente OH ou NH₂, on transforme un composé cyano de formule 10, au moyen d'hydroxylamine ou d'hydrazine, en le composé hydroxyamidine et/ou amidrazone correspondant ; et
(e) éventuellement, on transforme un composé obtenu de formule I en un sel d'addition d'acide et/ou on transforme un sel d'addition d'acide obtenu en le composé correspondant de formule I ou en un autre sel d'addition d'acide.

21. Composés de formule 10 telle que définie dans la revendication 20.
